(19) Europäisches Patentamt
European Patent Office
Office européen des brevets

(11) EP 0 709 380 A2

(12) EUROPÄISCHE PATENTANMELDUNG

(43) Veröffentlichungstag:
**01.05.1996 Patentblatt 1996/18**

(21) Anmeldenummer: **95116137.1**

(22) Anmeldetag: **13.10.1995**

(51) Int. Cl.[6]: **C07D 263/58**, C07D 285/08, C07D 285/12, C07D 277/68, A01N 43/836, A01N 43/82

(84) Benannte Vertragsstaaten:
**BE CH DE ES FR GB IT LI NL**

(30) Priorität: **26.10.1994 DE 4438178**

(71) Anmelder: **BAYER AG**
**D-51368 Leverkusen (DE)**

(72) Erfinder:
- **Förster, Heinz, Dr.**
  **D-56337 Kadenbach (DE)**
- **Lantzsch, Reinhard, Dr.**
  **D-42115 Wuppertal (DE)**
- **Santel, Hans-Joachim, Dr.**
  **D-51371 Leverkusen (DE)**
- **Dollinger, Markus, Dr.**
  **D-51381 Leverkusen (DE)**

# (54) N-methyl-N-isopropyl-heteroaryloxyacetamide

(57) Die Erfindung betrifft neue N-Methyl-N-isopropyl-heteroaryloxyacetamide der Formel (I),

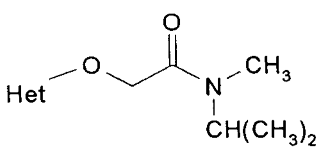

(I)

in welcher

Het für gegebenenfalls benzanelliertes und gegebenenfalls substituiertes Heteroaryl aus der Reihe Oxazolyl, Isoxazolyl, Thiazolyl, Isothiazolyl, Benzoxazolyl, Benzthiazolyl, 1,2,4-Oxadiazolyl, 1,3,4-Oxadiazolyl, 1,2,4-Thiadiazolyl und 1,3,4-Thiadiazolyl steht,

ein Verfahren zu ihrer Herstellung und ihre Verwendung als Herbizide.

EP 0 709 380 A2

Printed by Rank Xerox (UK) Business Services
2.11.3/3.4

## Beschreibung

Die Erfindung betrifft neue N-Methyl-N-isopropyl-heteroaryloxyacetamide, ein Verfahren zu ihrer Herstellung und ihre Verwendung als Herbizide.

Es ist bekannt, daß bestimmte N-Methyl-N-isopropyl-heterocyclyloxyacetamide, wie z.B. die Verbindung N-Methyl-N-isopropyl-2-(4-phenyl-1,2,3-thiadiazol-5-yl-oxy)-acetamid, herbizide Eigenschaften aufweisen (vgl. JP-A 03081269 - zitiert in Chem. Abstracts. 115:92307z). Die Wirkung dieser bekannten Verbindung(en) ist jedoch, insbesondere bei niedrigen Aufwandmengen und Wirkstoffkonzentrationen, nicht immer ganz zufriedenstellend.

Es wurden nun die neuen N-Methyl-N-isopropyl-heteroaryloxyacetamide der allgemeinen Formel (I) gefunden,

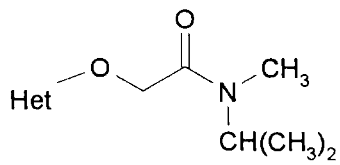 (I)

in welcher

Het für gegebenenfalls benzanelliertes und gegebenenfalls substituiertes Heteroaryl aus der Reihe Oxazolyl, Isoxazolyl, Thiazolyl, Isothiazolyl, Benzoxazolyl, Benzthiazolyl, 1,2,4-Oxadiazolyl, 1,3,4-Oxadiazolyl, 1,2,4-Thiadiazolyl und 1,3,4-Thiadiazolyl steht.

Weiter wurde gefunden, daß man die neuen N-Methyl-N-isopropyl-heteroaryloxyacetamide der Formel (I) erhält, wenn man substituierte Heteroarene der allgemeinen Formel (II)

Het-X (II)

in welcher

Het die oben angegebene Bedeutung hat und

X für Halogen, Alkylthio, Alkylsulfinyl oder Alkylsulfonyl steht,

mit N-Methyl-N-isopropyl-hydroxyacetamid der Formel (III)

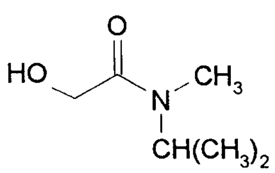 (III)

gegebenenfalls in Gegenwart eines Verdünnungsmittels und gegebenenfalls in Gegenwart eines Reaktionshilfsmittels umsetzt.

Schließlich wurde gefunden, daß die neuen N-Methyl-N-isopropyl-heteroaryloxyacetamide der allgemeinen Formel (I) interessante herbizide Eigenschaften besitzen.

Gegenstand der Erfindung sind vorzugsweise Verbindungen der Formel (I), in welcher

Het für gegebenenfalls benzanelliertes und gegebenenfalls substituiertes Heteroaryl aus der Reihe Oxazolyl, Isoxazolyl, Thiazolyl, Isothiazolyl, Benzoxazolyl, Benzthiazolyl, 1,2,4-Oxadiazolyl, 1,3,4-Oxadiazolyl, 1,2,4-Thiadiazolyl und 1,3,4-Thiadiazolyl steht, wobei die möglichen Substituenten vorzugsweise ausgewählt sind aus der Reihe Halogen, Cyano, (jeweils gegebenenfalls durch Fluor und/oder Chlor substituiertes) $C_1$-$C_6$-Alkyl, $C_2$-$C_6$-Alkenyl, $C_2$-$C_6$-Alkinyl, $C_1$-$C_6$-Alkoxy, $C_1$-$C_6$-Alkylthio, $C_1$-$C_6$-Alkylsulfinyl, $C_1$-$C_6$-Alkylsulfonyl, $C_1$-$C_6$-Alkylsulfonyl-methyl, $C_3$-$C_6$-Cycloalkyl, Phenyl, Phenoxymethyl, Benzylsulfonyl, Pyridyl, Furyl oder Thienyl.

Die Erfindung betrifft insbesondere Verbindungen der Formel (I), in welcher

Het für gegebenenfalls benzanelliertes und gegebenenfalls substituiertes Heteroaryl aus der Reihe 1,3-Oxazol-2-yl, 1,3-Thiazol-2-yl, Benzoxazol-2-yl, Benzthiazol-2-yl, 1,2,4-Thiadiazol-5-yl und 1,3,4-Thiadiazol-2-yl steht, wobei die möglichen Substituenten vorzugsweise ausgewählt sind aus der Reihe Fluor, Chlor, Brom, Cyano, (jeweils gegebenenfalls durch Fluor und/oder Chlor substituiertes) Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, n- oder i-Propylsulfinyl, Methylsulfonyl, Ethylsulfonyl, n- oder i-Propylsulfonyl, Cyclopropyl, Cyclobutyl, Cyclopentyl, Cyclohexyl, Phenyl, Pyridyl, Furyl oder Thienyl.

Die oben aufgeführten allgemeinen oder in Vorzugsbereichen angegebenen Restedefinitionen gelten sowohl für die Endprodukte der Formel (I) als auch entsprechend für die jeweils zu Herstellung benötigten Ausgangsstoffe bzw. Zwischenprodukte. Diese Restedefinitionen können untereinander, also auch zwischen den angegebenen Bereichen bevorzugter Verbindungen, beliebig kombiniert werden.

Verwendet man beispielsweise 2,4,5-Trichlor-thiazol und N-Methyl-N-isopropylhydroxyacetamid als Ausgangsstoffe, so kann der Reaktionsablauf beim erfindungsgemäßen Verfahren durch das folgende Formelschema skizziert werden:

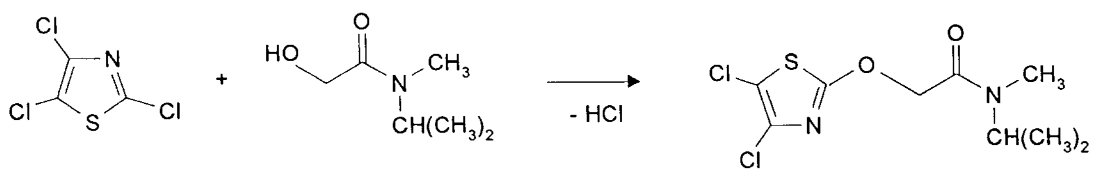


Die beim erfindungsgemäßen Verfahren zur Herstellung der Verbindungen der allgemeinen Formel (I) als Ausgangsstoffe zu verwendenden substituierten Heteroarene sind durch die Formel (II) allgemein definiert. In der Formel (II) hat Het vorzugsweise bzw. insbesondere diejenige Bedeutung, die bereits oben im Zusammenhang mit der Beschreibung der Verbindungen der Formel (I) als bevorzugt bzw. als insbesondere bevorzugt für Het angegeben wurde; X steht vorzugsweise für Fluor, Chlor, Brom, $C_1$-$C_4$-Alkylthio, $C_1$-$C_4$-Alkylsulfinyl oder $C_1$-$C_4$-Alkylsulfonyl, insbesondere für Chlor oder Methylsulfonyl.

Die Ausgangsstoffe der Formel (II) sind bekannt und/oder können nach an sich bekannten Verfahren hergestellt werden (vgl. J. Org. Chem. 27 (1962), 2589-2592; EP-A 5501; EP-A 18497; EP-A 165537; EP-A 308740; EP-A 348735; EP-A 348737).

Die beim erfindungsgemäßen Verfahren weiter als Ausgangsstoff zu verwendende Verbindung N-Methyl-N-isopropyl-hydroxyacetamid der Formel (III) ist bereits bekannt (vgl. EP-A 5501; EP-A 18497; Herstellungsbeispiele).

Als Verdünnungsmittel zur Durchführung des erfindungsgemäßen Verfahrens kommen die üblichen organischen Lösungsmittel in Betracht. Hierzu gehören insbesondere aliphatische, alicyclische oder aromatische, gegebenenfalls halogenierte Kohlenwasserstoffe, wie beispielsweise Pentan, Hexan, Heptan, Petrolether, Ligroin, Benzin, Benzol, Toluol, Xylol, Chlorbenzol, Dichlorbenzol, Cyclohexan, Methylcyclohexan, Dichlormethan, Chloroform, Tetrachlormethan; Ether, wie Diethylether, Diisopropylether, t-Butyl-methylether, t-Pentyl-methylether, Dioxan, Tetrahydrofuran, Ethylenglykol-dimethyl- oder -diethylether, Diethylenglykoldimethylether oder -diethylether; Ketone, wie Aceton, Butanon, Methyl-isopropylketon oder Methyl-isobutyl-keton; Nitrile, wie Acetonitril, Propionitril, Butyronitril oder Benzonitril; Amide, wie N,N-Dimethylformamid, N,N-Dimethylacetamid, N-Methylformanilid, N-Methyl-pyrrolidon oder Hexamethylphosphorsäuretriamid; Ester wie Essigsäure-methylester, -ethylester, -n- oder -i-propylester, -n-, -i- oder - s-butylester, Sulfoxide, wie Dimethylsulfoxid; Alkohole, wie Methanol, Ethanol, n- oder i-Propanol, n-, i-, s- oder t-Butanol, Ethylenglykol-monomethylether oder - monoethylether, Diethylenglykolmonomethylether, Diethylenglykolmonoethylether; deren Gemische mit Wasser oder reines Wasser.

Das erfindungsgemäße Verfahren wird vorzugsweise in Gegenwart eines geeigneten Reaktionshilfsmittels durchgeführt. Als solche kommen alle üblichen anorganischen oder organischen Basen infrage. Hierzu gehören beispielsweise Alkalimetall- oder Erdalkalimetall-hydride, -hydroxide, -amide, -alkoholate, -acetate, -carbonate oder -hydrogencarbonate, wie beispielsweise Lithium-, Natrium-, Kalium- oder Calcium-hydrid, Lithium-, Natrium- oder Kalium-amid, Natrium- oder Kalium-methylat, Natrium- oder Kalium-ethylat, Natrium- oder Kalium-propylat, Aluminiumisopropylat, Natrium- oder Kalium-tert-butylat, Natrium- oder Kalium-hydroxid, Ammoniumhydroxid, Natrium-, Kalium- oder Calcium-acetat, Ammoniumacetat, Natrium-, Kalium- oder Calcium-carbonat, Ammoniumcarbonat, Natrium- oder Kalium-hydrogencarbonat, sowie basische organische Stickstoffverbindungen, wie Trimethylamin, Triethylamin, Tripropylamin, Tributylamin, Ethyldiisopropylamin, N,N-Dimethylcyclohexylamin, Dicyclohexylamin, Ethyl-dicyclohexylamin, N,N-Dimethylanilin, N,N-Dimethyl-benzylamin, Pyridin, 2-Methyl-, 3-Methyl- und 4-Methyl-pyridin, 2,4-Dimethyl-, 2,6-Dimethyl-, 3,4-Dimethyl- und 3,5-Dimethyl-pyridin, 5-Ethyl-2-methyl-pyridin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens in einem größeren Bereich variiert werden. Im allgemeinen arbeitet man bei Temperaturen zwischen -50°C und +150°C, vorzugsweise bei Temperaturen zwischen -30°C und +100°C, insbesondere bei Temperaturen zwischen -10°C und +50°C.

Das erfindungsgemäße Verfahren wird im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Zur Durchführung des erfindungsgemäßen Verfahrens werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Reaktionshilfsmittels durchgeführt, und das Reaktionsgemisch wird mehrere Stunden bei der jeweils erforderlichen Temperatur gerührt. Die Aufarbeitung erfolgt bei dem erfindungsgemäßen Verfahren jeweils nach üblichen Methoden (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium. Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen und dikotylen Unkräutern in monokotylen und dikotylen Kulturen, vor allem im Vorauflauf-Verfahren.

In gewissem Umfang zeigen die Verbindungen der Formel (I) auch Wirkung gegen Blattinsekten und fungizide Wirkung, beispielsweise gegen Pyricularia oryzae an Reis.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methyl-ethylketon, Methyl-isobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate,

Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.
Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4-D, 2,4-DB, 2,4-DP, Fluroxypyr, MCPA, MCPP und Triclopyr; Aryloxy-phenoxyalkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 10 g und 10 kg Wirkstoff pro Hektar Bodenfläche, vorzugsweise zwischen 50 g und 5 kg pro ha.

Die Herstellung und die Verwendung der erfindungsgemäßen Wirkstoffe geht aus den nachfolgenden Beispielen hervor.

Herstellungsbeispiele:

Beispiel 1

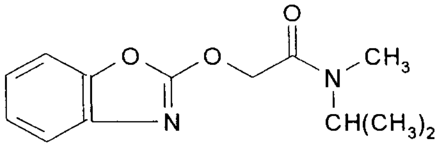

3,0 g (20 mMol) 2-Chlor-benzoxazol werden unter Rühren bei 0°C bis +5°C zu einer Mischung aus 2,7 g (20 mMol) N-Methyl-N-isopropyl-hydroxyacetamid, 2,2, g (20 mMol) Kalium-t-butylat, 15 ml Acetonitril und 50 ml t-Butanol gegeben.

Die Reaktionsmischung wird dann 12 Stunden bei 20°C gerührt, anschließend mit Wasser langsam auf etwa das doppelte Volumen verdünnt und mit Eis abgekühlt. Das hierbei kristallin angefallene Produkt wird durch Absaugen isoliert.

Man erhält 4,0 g (82% der Theorie) N-Methyl-N-isopropyl-2-(benzoxazol-2-yl-oxy)-acetamid vom Schmelzpunkt 56°C.

Analog Beispiel 1 sowie entsprechend der allgemeinen Beschreibung des erfindungsgemäßen Herstellungsverfahrens können beispielsweise auch die in der nachstehenden Tabelle 1 aufgeführten Verbindungen der Formel (I) hergestellt werden.

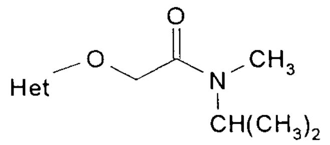

(I)

Tabelle 1: Beispiele für die Verbindungen der Formel (I)

| Bsp.-Nr. | Het | Physikal. Daten |
|---|---|---|
| 2 | 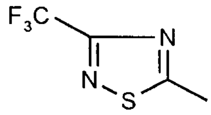 | $n_D^{20}$ = 1,4662 |
| 3 | 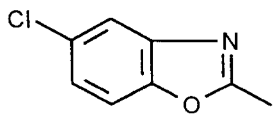 | Fp.: 74°C |
| 4 | 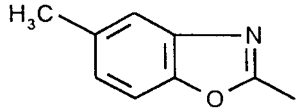 | Fp.: 106°C |
| 5 | 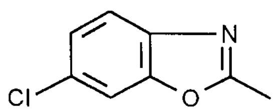 | Fp.: 98°C |
| 6 | 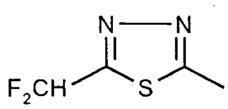 | $n_D^{20}$ = 1,4902 |
| 7 | 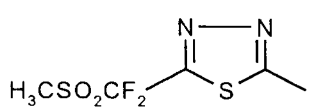 | $n_D^{20}$ = 1,4872 |
| 8 | 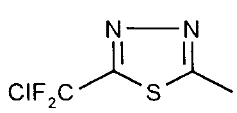 | $n_D^{20}$ = 1,4792 |
| 9 | 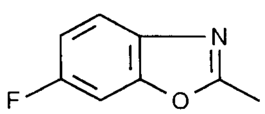 | Fp.: 75°C |

Tabelle 1 (Fortsetzung)

| Bsp.-Nr. | Het | Physikal. Daten |
|---|---|---|
| 10 | 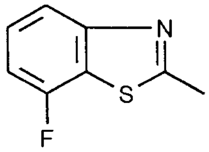 | $n_D^{20}$ = 1,5288 |
| 11 | 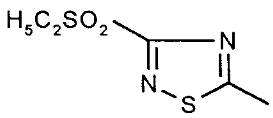 | $n_D^{20}$ = 1,5192 |
| 12 | 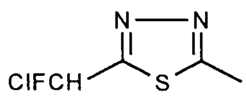 | (Öl) |
| 13 | 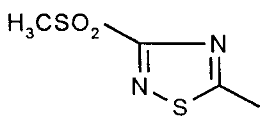 | $n_D^{20}$ = 1,5075 |
| 14 | 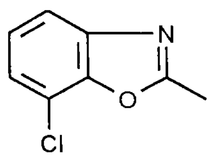 | Fp.: 90°C |
| 15 | 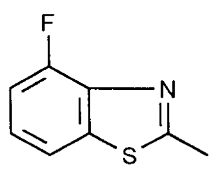 | Fp.: 76°C |
| 16 | 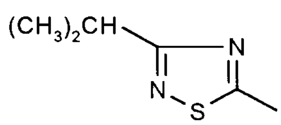 | $n_D^{20}$ = 1,4380 |
| 17 | 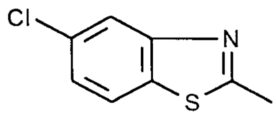 | Fp.: 85°C |

Tabelle 1 (Fortsetzung)

| Bsp.-Nr. | Het | Physikal. Daten |
|---|---|---|
| 18 | 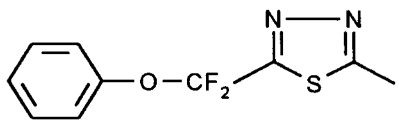 | $n_D^{20}$ = 1,5099 |
| 19 | 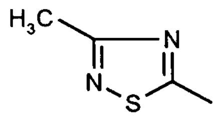 | $n_D^{20}$ = 1,4885 |
| 20 | 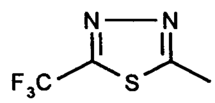 | $n_D^{20}$ = 1,4665 |
| 21 | 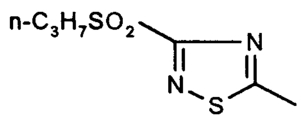 | $n_D^{20}$ = 1,5249 |
| 22 | 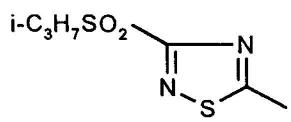 | $n_D^{20}$ = 1,5189 |
| 23 | 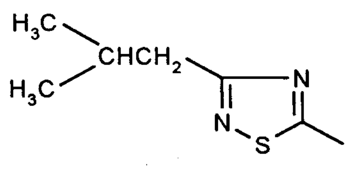 | |
| 24 | 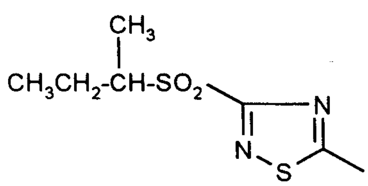 | $n_D^{20}$ = 1,5046 |
| 25 | 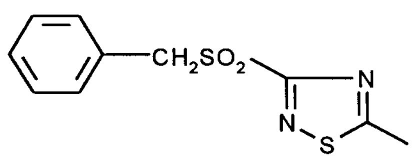 | |
| 26 | 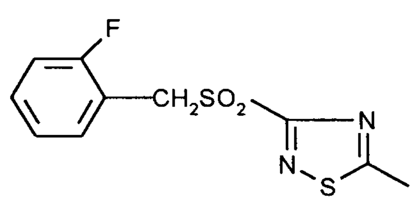 | |

Ausgangsverbindung der Formel (III):

Stufe 1

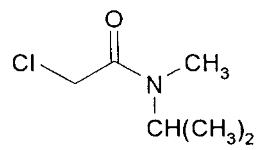

Zu einer Mischung aus 30 g (0,40 Mol) N-Methylisopropylamin, 300 ml Methylenchlorid, 120 ml Wasser und 55 g (0,40 Mol) Kaliumcarbonat werden bei 0°C bis +5°C langsam unter Rühren 45 g (0,40 Mol) Chloracetylchlorid gegeben. Die Reaktionsmischung wird 20 Stunden bei 20°C gerührt, dann mit Wasser auf etwa das doppelte Volumen verdünnt und durchgeschüttelt. Die organische Phase wird dann abgetrennt, mit Wasser gewaschen, mit Natriumsulfat getrocknet und filtriert. Vom Filtrat wird das Lösungsmittel im Wasserstrahlvakuum sorgfältig abdestilliert.

Man erhält 54 g (91% der Theorie) N-Methyl-N-isopropyl-chloracetamid als öligen Rückstand vom Brechungsindex $n_D^{20}$ = 1,4700.

Stufe 2

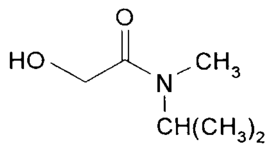

Eine Mischung aus 52 g (0,35 Mol) N-Methyl-N-isopropyl-chloracetamid, 10,4 g (0,07 Mol) Kaliumcarbonat und 0,4 ml Triethylamin wird auf 110°C erhitzt und bei dieser Temperatur unter Rühren mit 36 g (0,44 Mol) Natriumacetat portionsweise versetzt. Die Mischung wird dann noch eine Stunde bei 120°C gerührt und anschließend bei 50°C bis 60°C mit 200 ml Methanol versetzt. Dann wird 4 Stunden unter Rückfluß zum Sieden erhitzt und anschließend eingeengt. Der Rückstand wird mit Wasser/Methylenchlorid geschüttelt, die organische Phase mit Magnesiumsulfat getrocknet und filtriert. Vom Filtrat wird das Lösungsmittel im Wasserstrahlvakuum sorgfältig abdestilliert.

Man erhält 37 g (81% der Theorie) N-Methyl-N-isopropyl-hydroxyacetamid als öligen Rückstand vom Brechungsindex $n_D^{20}$ = 1,4572.

Anwendungsbeispiele:

Beispiel A

Pre-emergence-Test

| | |
|---|---|
| Lösungsmittel: | 5 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät. Nach 24 Stunden wird der Boden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)

100 % = totale Vernichtung

In diesem Test zeigen beispielsweise die Verbindungen gemäß den Herstellungsbeispielen 2, 5, 6, 8, 11, 12, 13, 20, 21

und 24 bei sehr guter Verträglichkeit gegenüber Kulturpflanzen, wie z.B. Baumwolle oder Gerste, starke Wirkung gegen Unkräuter.

## Patentansprüche

1. N-Methyl-N-isopropyl-heteroaryloxyacetamide der allgemeinen Formel (I),

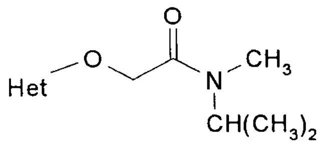

(I)

in welcher

Het für gegebenenfalls benzanelliertes und gegebenenfalls substituiertes Heteroaryl aus der Reihe Oxazolyl, Isoxazolyl, Thiazolyl, Isothiazolyl, Benzoxazolyl, Benzthiazolyl, 1,2,4-Oxadiazolyl, 1,3,4-Oxadiazolyl, 1,2,4-Thiadiazolyl und 1,3,4-Thiadiazolyl steht.

2. Verbindungen der Formel (I) gemäß Anspruch 1, dadurch gekennzeichnet, daß darin

Het für gegebenenfalls benzanelliertes und gegebenenfalls substituiertes Heteroaryl aus der Reihe Oxazolyl, Isoxazolyl, Thiazolyl, Isothiazolyl, Benzoxazolyl, Benzthiazolyl, 1,2,4-Oxadiazolyl, 1,3,4-Oxadiazolyl, 1,2,4-Thiadiazolyl und 1,3,4-Thiadiazolyl steht, wobei die möglichen Substituenten ausgewählt sind aus der Reihe Halogen, Cyano, (jeweils gegebenenfalls durch Fluor und/oder Chlor substituiertes) $C_1$-$C_6$-Alkyl, $C_2$-$C_6$-Alkenyl, $C_2$-$C_6$-Alkinyl, $C_1$-$C_6$-Alkoxy, $C_1$-$C_6$-Alkylthio, $C_1$-$C_6$-Alkylsulfinyl, $C_1$-$C_6$-Alkylsulfonyl, $C_1$-$C_6$-Alkylsulfonyl-methyl, $C_3$-$C_6$-Cycloalkyl, Phenyl, Phenoxymethyl, Benzylsulfonyl, Pyridyl, Furyl oder Thienyl.

3. Verbindungen der Formel (I) gemäß Anspruch 1, dadurch gekennzeichnet, daß darin

Het für gegebenenfalls benzanelliertes und gegebenenfalls substituiertes Heteroaryl aus der Reihe 1,3-Oxazol-2-yl, 1,3-Thiazol-2-yl, Benzoxazol-2-yl, Benzthiazol-2-yl, 1,2,4-Thiadiazol-5-yl und 1,3,4-Thiadiazol-2-yl steht, wobei die möglichen Substituenten ausgewählt sind aus der Reihe Fluor, Chlor, Brom, Cyano, (jeweils gegebenenfalls durch Fluor und/oder Chlor substituiertes) Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, n- oder i-Propylsulfinyl, Methylsulfonyl, Ethylsulfonyl, n- oder i-Propylsulfonyl, Cyclopropyl, Cyclobutyl, Cyclopentyl, Cyclohexyl, Phenyl, Pyridyl, Furyl oder Thienyl.

4. Verfahren zur Herstellung von N-Methyl-N-isopropyl-heteroaryloxyacetamiden der Formel (I) gemäß Anspruch 1, dadurch gekennzeichnet, daß man substituierte Heteroarene der Formel (II),

$$\text{Het-X} \qquad \text{(II)}$$

in welcher

Het die in Anspruch 1 angegebene Bedeutung hat und

X für Halogen, Alkylthio, Alkylsulfinyl oder Alkylsulfonyl steht,

mit N-Methyl-N-isopropyl-hydroxyacetamid der Formel (III)

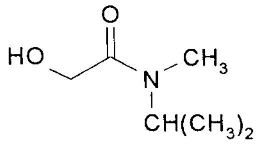

(III)

gegebenenfalls in Gegenwart eines Verdünnungsmittels und gegebenenfalls in Gegenwart eines Reaktionshilfsmittels umsetzt.

**5.** Herbizide Mittel, gekennzeichnet durch einen Gehalt an mindestens einer Verbindung der Formel (I) gemäß Anspruch 1.

**6.** Verwendung von Verbindungen der allgemeinen Formel (I) gemäß Anspruch 1 zur Bekämpfung von unerwünschtem Pflanzenwachstum.

**7.** Verfahren zur Bekämpfung von Unkräutern, dadurch gekennzeichnet, daß man Verbindungen der allgemeinen Formel (I) gemäß Anspruch 1 auf die Unkräuter oder ihren Lebensraum einwirken läßt.

**8.** Verfahren zur Herstellung von herbiziden Mitteln, dadurch gekennzeichnet, daß man Verbindungen der allgemeinen Formel (I) gemäß Anspruch 1 mit Streckmitteln und/oder oberflächenaktiven Mitteln vermischt.